# EUROPEAN PATENT APPLICATION

(11) **EP 3 919 062 A1**
(43) Date of publication of application: **08.12.2021**
(21) Application number: 20305578.5
(22) Date of filing: 02.06.2020
(51) Int. Cl.: A61K 31/7084, A61K 31/675, A61K 31/7072, A61P 11/00, A61P 31/14

(54) **MODULATORS OF PURINERGIC RECEPTORS AND RELATED IMMUNE CHECKPOINT FOR TREATING ACUTE RESPIRATORY DISTRESS SYNDROM**

(71) Applicant: Institut Gustave-Roussy, 94800 Villejuif (FR)
(72) Inventor: The designation of the inventor has not yet been filed
(74) Representative: Regimbeau

(57) **Abstract**

The inventors herein show that purinergic receptors regulate the conversion of macrophage pro-inflammatory reprogramming into anti-inflammatory phenotype in patients suffering from COVID-19 disease. Moreover, they show that P2Y receptor agonists repress NLRP3 inflammasome-dependent IL-1b secretion, but also impair the replication and the cytopathogenic effects of SARS-CoV-2. These results therefore suggest that some purinergic receptors agonists can treat acute lung injury and respiratory disease that are associated with SARS-CoV-2 infection. In addition, their results show that antagonists of the purinergic receptors P2X impair the replication of said virus. The present invention therefore proposes to use purinergic receptors modulators and NLR3-P2Y2R immune checkpoint modulators to treat patients suffering from a virus-induced acute respiratory distress syndrome.

SEQUENCE LISTING

<110>  INSTITUT GUSTAVE ROUSSY

<120>  MODULATORS OF PURINERGIC RECEPTORS AND RELATED IMMUNE CHECKPOINT
       FOR TREATING ACUTE RESPIRATORY DISTRESS SYNDROM

<130>  B76136 D40664

<160>  8

<170>  PatentIn version 3.5

<210>  1
<211>  377
<212>  PRT
<213>  homo sapiens

<400>  1

Met Ala Ala Asp Leu Gly Pro Trp Asn Asp Thr Ile Asn Gly Thr Trp
1               5                   10                  15

Asp Gly Asp Glu Leu Gly Tyr Arg Cys Arg Phe Asn Glu Asp Phe Lys
            20                  25                  30

Tyr Val Leu Leu Pro Val Ser Tyr Gly Val Val Cys Val Pro Gly Leu
        35                  40                  45

Cys Leu Asn Ala Val Ala Leu Tyr Ile Phe Leu Cys Arg Leu Lys Thr
    50                  55                  60

Trp Asn Ala Ser Thr Thr Tyr Met Phe His Leu Ala Val Ser Asp Ala
65                  70                  75                  80

Leu Tyr Ala Ala Ser Leu Pro Leu Leu Val Tyr Tyr Tyr Ala Arg Gly
                85                  90                  95

Asp His Trp Pro Phe Ser Thr Val Leu Cys Lys Leu Val Arg Phe Leu
            100                 105                 110

Phe Tyr Thr Asn Leu Tyr Cys Ser Ile Leu Phe Leu Thr Cys Ile Ser
        115                 120                 125

Val His Arg Cys Leu Gly Val Leu Arg Pro Leu Arg Ser Leu Arg Trp
    130                 135                 140

Gly Arg Ala Arg Tyr Ala Arg Arg Val Ala Gly Ala Val Trp Val Leu
145                 150                 155                 160

Val Leu Ala Cys Gln Ala Pro Val Leu Tyr Phe Val Thr Thr Ser Ala
                165                 170                 175

Arg Gly Gly Arg Val Thr Cys His Asp Thr Ser Ala Pro Glu Leu Phe
            180                 185                 190

Ser Arg Phe Val Ala Tyr Ser Ser Val Met Leu Gly Leu Leu Phe Ala
        195                 200                 205

Val Pro Phe Ala Val Ile Leu Val Cys Tyr Val Leu Met Ala Arg Arg
    210                 215                 220

Leu Leu Lys Pro Ala Tyr Gly Thr Ser Gly Gly Leu Pro Arg Ala Lys
225                 230                 235                 240

Arg Lys Ser Val Arg Thr Ile Ala Val Val Leu Ala Val Phe Ala Leu
                245                 250                 255

Cys Phe Leu Pro Phe His Val Thr Arg Thr Leu Tyr Tyr Ser Phe Arg
            260                 265                 270

Ser Leu Asp Leu Ser Cys His Thr Leu Asn Ala Ile Asn Met Ala Tyr
        275                 280                 285

Lys Val Thr Arg Pro Leu Ala Ser Ala Asn Ser Cys Leu Asp Pro Val
    290                 295                 300

Leu Tyr Phe Leu Ala Gly Gln Arg Leu Val Arg Phe Ala Arg Asp Ala
305                 310                 315                 320

Lys Pro Pro Thr Gly Pro Ser Pro Ala Thr Pro Ala Arg Arg Arg Leu
                325                 330                 335

Gly Leu Arg Arg Ser Asp Arg Thr Asp Met Gln Arg Ile Glu Asp Val
            340                 345                 350

Leu Gly Ser Ser Glu Asp Ser Arg Arg Thr Glu Ser Thr Pro Ala Gly
        355                 360                 365

Ser Glu Asn Thr Lys Asp Ile Arg Leu
    370                 375

<210> 2
<211> 595
<212> PRT
<213> homo sapiens

<400> 2

Met Pro Ala Cys Cys Ser Cys Ser Asp Val Phe Gln Tyr Glu Thr Asn
1               5                   10                  15

Lys Val Thr Arg Ile Gln Ser Met Asn Tyr Gly Thr Ile Lys Trp Phe
            20                  25                  30

Phe His Val Ile Ile Phe Ser Tyr Val Cys Phe Ala Leu Val Ser Asp
        35                  40                  45

Lys Leu Tyr Gln Arg Lys Glu Pro Val Ile Ser Ser Val His Thr Lys
    50                  55                  60

Val Lys Gly Ile Ala Glu Val Lys Glu Glu Ile Val Glu Asn Gly Val
65                  70                  75                  80

Lys Lys Leu Val His Ser Val Phe Asp Thr Ala Asp Tyr Thr Phe Pro
                85                  90                  95

Leu Gln Gly Asn Ser Phe Phe Val Met Thr Asn Phe Leu Lys Thr Glu
            100                 105                 110

Gly Gln Glu Gln Arg Leu Cys Pro Glu Tyr Pro Thr Arg Arg Thr Leu
        115                 120                 125

Cys Ser Ser Asp Arg Gly Cys Lys Lys Gly Trp Met Asp Pro Gln Ser
    130                 135                 140

Lys Gly Ile Gln Thr Gly Arg Cys Val Val Tyr Glu Gly Asn Gln Lys
145                 150                 155                 160

Thr Cys Glu Val Ser Ala Trp Cys Pro Ile Glu Ala Val Glu Glu Ala
                165                 170                 175

Pro Arg Pro Ala Leu Leu Asn Ser Ala Glu Asn Phe Thr Val Leu Ile
            180                 185                 190

Lys Asn Asn Ile Asp Phe Pro Gly His Asn Tyr Thr Thr Arg Asn Ile
        195                 200                 205

Leu Pro Gly Leu Asn Ile Thr Cys Thr Phe His Lys Thr Gln Asn Pro
    210                 215                 220

Gln Cys Pro Ile Phe Arg Leu Gly Asp Ile Phe Arg Glu Thr Gly Asp
225                 230                 235                 240

Asn Phe Ser Asp Val Ala Ile Gln Gly Gly Ile Met Gly Ile Glu Ile
                245                 250                 255

Tyr Trp Asp Cys Asn Leu Asp Arg Trp Phe His His Cys Arg Pro Lys
            260                 265                 270

Tyr Ser Phe Arg Arg Leu Asp Asp Lys Thr Thr Asn Val Ser Leu Tyr
        275                 280                 285

Pro Gly Tyr Asn Phe Arg Tyr Ala Lys Tyr Tyr Lys Glu Asn Asn Val
    290                 295                 300

Glu Lys Arg Thr Leu Ile Lys Val Phe Gly Ile Arg Phe Asp Ile Leu
305                 310                 315                 320

Val Phe Gly Thr Gly Gly Lys Phe Asp Ile Ile Gln Leu Val Val Tyr
                325                 330                 335

Ile Gly Ser Thr Leu Ser Tyr Phe Gly Leu Ala Ala Val Phe Ile Asp
            340                 345                 350

Phe Leu Ile Asp Thr Tyr Ser Ser Asn Cys Cys Arg Ser His Ile Tyr
        355                 360                 365

Pro Trp Cys Lys Cys Cys Gln Pro Cys Val Val Asn Glu Tyr Tyr Tyr
    370                 375                 380

Arg Lys Lys Cys Glu Ser Ile Val Glu Pro Lys Pro Thr Leu Lys Tyr
385                 390                 395                 400

Val Ser Phe Val Asp Glu Ser His Ile Arg Met Val Asn Gln Gln Leu
                405                 410                 415

Leu Gly Arg Ser Leu Gln Asp Val Lys Gly Gln Glu Val Pro Arg Pro
            420                 425                 430

Ala Met Asp Phe Thr Asp Leu Ser Arg Leu Pro Leu Ala Leu His Asp
        435                 440                 445

Thr Pro Pro Ile Pro Gly Gln Pro Glu Glu Ile Gln Leu Leu Arg Lys
    450                 455                 460

Glu Ala Thr Pro Arg Ser Arg Asp Ser Pro Val Trp Cys Gln Cys Gly
465                 470                 475                 480

Ser Cys Leu Pro Ser Gln Leu Pro Glu Ser His Arg Cys Leu Glu Glu
                485                 490                 495

Leu Cys Cys Arg Lys Lys Pro Gly Ala Cys Ile Thr Thr Ser Glu Leu
            500                 505                 510

Phe Arg Lys Leu Val Leu Ser Arg His Val Leu Gln Phe Leu Leu Leu

515                 520                 525

Tyr Gln Glu Pro Leu Leu Ala Leu Asp Val Asp Ser Thr Asn Ser Arg
    530                 535                 540

Leu Arg His Cys Ala Tyr Arg Cys Tyr Ala Thr Trp Arg Phe Gly Ser
545                 550                 555                 560

Gln Asp Met Ala Asp Phe Ala Ile Leu Pro Ser Cys Cys Arg Trp Arg
                565                 570                 575

Ile Arg Lys Glu Phe Pro Lys Ser Glu Gly Gln Tyr Ser Gly Phe Lys
            580                 585                 590

Ser Pro Tyr
        595

<210>  3
<211>  22
<212>  DNA
<213>  artificial

<220>
<223>  RdRp SARSr-F

<400>  3
gtgaratggt catgtgtggc gg                                                22

<210>  4
<211>  26
<212>  DNA
<213>  artificial

<220>
<223>  RdRp SARSr-R

<400>  4
caratgttaa asacactatt agcata                                            26

<210>  5
<211>  25
<212>  DNA
<213>  artificial

<220>
<223>  RdRp SARSr-P2

<400>  5
caggtggaac ctcatcagga gatgc                                             25

<210>  6
<211>  26
<212>  DNA
<213>  artificial

<220>
<223>  E Sarbeco P1

<400>  6
acactagcca tccttactgc gcttcg                                          26


<210>  7
<211>  26
<212>  DNA
<213>  artificial

<220>
<223>  E Sarbeco F

<400>  7
acaggtacgt taatagttaa tagcgt                                          26


<210>  8
<211>  22
<212>  DNA
<213>  artificial

<220>
<223>  E Sarbeco R

<400>  8
atattgcagc agtacgcaca ca                                              22

## Description

### SUMMARY OF THE INVENTION

The inventors herein show that purinergic receptors regulate the conversion of macrophage pro-inflammatory reprogramming into anti-inflammatory phenotype in patients suffering from COVID-19 disease. Moreover, they show that P2Y receptor agonists repress NLRP3 inflammasome-dependent IL-1b secretion, but also impair the replication and the cytopathogenic effects of SARS-CoV-2. These results therefore suggest that some purinergic receptors agonists can treat acute lung injury and respiratory disease that are associated with SARS-CoV-2 infection. In addition, their results show that antagonists of the purinergic receptors P2X impair the replication of said virus. The present invention therefore proposes to use purinergic receptors modulators and NLR3-P2Y2R immune checkpoint modulators to treat patients suffering from a virus-induced acute respiratory distress syndrome.

### BACKGROUND ART

Coronavirus disease 2019 (COVID-19) is a pandemic caused by a novel strain of β-coronavirus, severe acute respiratory syndrome-coronavirus 2 (SARS-CoV-2). While infection can be asymptomatic, especially in children and young adults, the most common symptoms of COVID-19 are fever and cough, with a majority of patients developing dyspnea, reflecting a tropism of the virus for the lung (Guan and Zhong, 2020). Most patients presenting with a mild disease develop an efficient immune response (Thevarajan et al., 2020) but some of them suddenly evolve into a severe form with acute respiratory distress syndrome needing admission in intensive care unit (ICU), indicative of critical condition, and often culminating into multi-organ dysfunction leading to death (Wang et al., 2020). SARS-CoV-2 belongs to the species *Coronavirus,* in the genus *Betacoronavirus* and family *Coronaviridae.*

In humans, coronavirus infections can cause respiratory pathologies associated with symptoms similar to the common cold, bronchiolitis and more serious diseases such as the Severe Acute Respiratory Syndrome caused by SARS-CoV-1, which generated an epidemic in 2003, and the Middle Eastern Respiratory Syndrome caused by MERS-CoV, which generated an epidemic in 2012. SARS-CoV-2 is the betacoronavirus causing the coronavirus epidemic of 2019-2020, generating the form of pneumonia known as coronavirus disease 2019 or COVID-19.

On 18 March 2020, there were more than 215,000 cases in 156 countries and 8,732 people had lost their lives (from Johns Hopkins CSSE and Dong et al., Lancet Infect. Dis (2020)). Despite the fact that disease severity (including oxygen saturation, respiratory rate, blood leukocyte/lymphocyte count and chest X-ray/CT manifestations) predicts poor clinical outcomes (Guan et al., MedRxiv (2020)), physio-pathological mechanisms responsible for COVID-19 remain unknown.

Symptoms of infection with SARS-CoV-2 are roughly similar to those of seasonal influenza infections: they include fever, fatigue, dry cough, shortness of breath, difficult breathing, pneumonia, renal failure, and may lead to death in severe cases. The severity of clinical signs requires that approximately 20% of patients remain in hospital and 5% require admission to intensive care. The most serious forms are observed in people who are vulnerable because of their age (over 70) or associated diseases such as hypertension, diabetes and/or coronary heart disease.

It is of the most importance to identify therapeutic compounds for preventing and/or treating COVID-19 disease. Indeed, today, no specific therapy or vaccine is currently available for the prevention or treatment of SARS-CoV-2 viral infection. The majority of treatments currently received by COVID-19 patients are primarily aimed at alleviating the symptoms of fever, cough and dyspnea in order to promote spontaneous recovery.

Antiviral compounds, known for their therapeutic activity on other types of viruses, are currently tested in clinical trials. The French High Council for Public Health issued an opinion on March 23, 2020 on the therapeutic recommendations for the management of COVID-19 disease. This official notice proposes in particular to use the following therapeutic compounds: Remdesivir, Lopinavir/Rotinavir and Hydroxychloroquine. Remdesivir has been initially developed for the treatment of Ebola virus infections. Remdesivir is a broad-spectrum antiviral compound, acting as a nucleoside analogue, specifically an adenosine analogue. Its presence misleads the viral polymerase and causes a reduction in viral RNA synthesis. Lopinavir is a viral protease inhibitor, previously used against the human immunodeficiency virus (HIV). Lopinavir inhibits the production of functional proteins by the new virions, thereby blocking the spread of the virus. Lopinavir is rapidly degraded in the body. For this reason, it is administered in fixed combination with Ritonavir, which inhibit cytochrome P450 monooxygenases, thereby slowing the degradation of Lopinavir by these enzymes. Hydroxychloroquine, initially known for its anti-malaria activity, has been shown to have an apparent efficacy in the treatment of Covid-19 (Yao et al., 2020). However, clinical data are still limited and controversial.

There is a pressing urgency to identify preventive and therapeutic treatments to impair the replication of the virus, or alleviate the symptoms of the COVID disease, in order to decrease its severity. In this context, identification of biological targets of the SARS-CoV-2 may allow to define novel therapeutic strategies.

Like SARS-CoV infection, SARS-CoV2 infection frequently leads to acute lung injury (ALI). Fatal acute respiratory disease (ARD) is the major complication in patients with severe disease (Wang et al., JAMA (2020); Fu et al., Virol Sinica (2020)). Most patients who died of ARD exhibited an acute onset of lung inflammation (Ware and Matthay, N Engl J Med (2000); Herold et al., Front Immunol (2011)), thus highlighting the urgent need to characterize molecular and cellular mechanisms responsible for virus-mediated lung inflammation.

The accumulation of pro-inflammatory macrophages in infected lungs has been extensively reported during SARS-CoV infection (Ware and Matthay, N Engl J Med (2000); Herold et al., Front Immunol (2011) and was proposed as a key event during SARS-CoV pathology (Liu et al., JCI Insight (2019)). Assuming that SARS-CoV-2 shares similar inflammatory responses with SARS-CoV, elucidating molecular mechanisms that drive the polarization of lung macrophages towards pro-inflammatory could lead to the identification of novel targets for treatment of SARS-CoV2 mediated-ALI and ARD.

An increased secretion of the pro-inflammatory cytokines such as interleukin (IL)-1β, IFN-Inducible protein-10 (IP-10), IL-4 and IL-6 was detected during SARS-CoV-2 infection (Huang et al., Lancet China (2020)) and aberrant inflammatory CD14⁺CD16⁺ monocytes were described in severe pulmonary syndrome patients (Zhou et al., BioRxiv (2020)). The pro-inflammatory functional reprogramming of macrophages is dependent of Fc-gamma receptor signaling and may activate NLRP3-dependent signaling pathways (Liu et al., JCI Insight (2019), Duffy et al., J Leuko Bio (2016)). Consistently, SARS-CoV open reading frame-8b was recently shown to trigger NLRP3 inflammasome activation (Shi et al., Cell Death Discover (2019)). Additionally, the transmembrane pore-forming viral Viroporin 3a (also known as SARS-COV 3a) was shown to activate the NLRP3 inflammasome in lipopolysaccharide (LPS)-primed macrophages (Chen et al., Front Microbiol (2019)).

It has been reported that the (NLR) protein 3 NLRP3 interacts with the purinergic receptor P2Y2 and dictates the early steps of HIV-1 infection (Paoletti et al., Cell Rep (2019)). More importantly, it was also shown that P2Y2 dictates NLRP3 degradation through ubiquitinylation (Paoletti et al., Cell Rep (2019)), thus revealing that P2Y2 acts as negative regulator of NLRP3 activation.

Yet, despite all these results, the role of the NLRP3-P2Y2 immune checkpoint in the immune response during SARS-CoV infections has never been studied so far. There is a need to evaluate if the modulation of NLRP3-P2Y2 immune checkpoint and especially the purinergic receptor signaling pathways could represent an innovative therapeutic strategy to avoid SARS-CoV-2-induced ARD.

### DETAILED DESCRIPTION OF THE INVENTION

### Importance of purinergic receptors and related immune checkpoint during the covid disease

Macrophages derived from monocyte precursors undergo specific differentiation depending on the local tissue environment. The various macrophage functions are linked to the type of receptor interaction on the macrophage and the presence of cytokines. Similar to the T helper type 1 and T helper type 2 (TH1-TH2) polarization, two distinct states of polarized activation for macrophages have been defined: the classically activated pro-inflammatory macrophage phenotype and the alternatively activated anti-inflammatory macrophage phenotype. Similar to T cells, there are some activating macrophages and some suppressive macrophages. Therefore, macrophages should be defined based on their specific functional activities. Classically activated pro-inflammatory macrophages have the role of effector cells in TH1 cellular immune responses, whereas the alternatively activated (M2) macrophages appear to be involved in immunosuppression and tissue repair.

Granulocyte macrophage colony stimulating factor (GM-CSF) and macrophage colony stimulating factor (M-CSF) are involved in the differentiation of monocytes to these two categories of macrophages : on a one hand, human GM-CSF polarize monocytes towards the pro-inflammatory macrophage subtype with a "pro-inflammatory" cytokine profile (e.g. TNF-alpha, IL-1beta, IL-6, IL-12 and IL-23); on another hand, treatment of monocytes with M-CSF induces macrophages into producing "anti-inflammatory" cytokines (e.g. IL-10, TGF-beta and IL-1ra) characteristic of M2 macrophages.

LPS and the TH1 cytokine IFN-gamma polarize macrophages towards the pro-inflammatory phenotype which induces the macrophage to produce large amounts of IL-1beta, TNF, IL-12, and IL-23. This helps to drive antigen specific TH1 and TH17 cell inflammatory responses forward and thus participates to the clearance of invading microorganisms. The antimicrobial functions of pro-inflammatory macrophages are linked to up-regulation of enzymes, such as inducible nitric oxide synthase (iNOS) that generates nitric oxide from L-arginine. The secretion of IL-6, IL-23, and IL-1beta are important factors in the induction and maintenance of Th17 cells. In some cases inflammatory responses can trigger tissue damage (toxic activity or reactive oxygen), resulting in an uncontrolled macrophage inflammatory response which could become pathogenic. For example, uncontrolled macrophage inflammatory response participates in the pathogenesis of inflammatory bowel disease (IBD).

By contrast, exposure of macrophages to the TH2 cytokine IL-4 produces a M2 phenotype which induces the production of high levels of IL-10 and IL-1RA and low expression of IL-12. These cells reduce inflammation, are immunoregulators, promote tissue remodeling and tumor progression. Indeed, recent studies indicate that tumor-associated macrophages (TAMs) show a anti-inflammatory phenotype. These tumor-associated macrophages (TAM) produce interleukin IL-10 and transforming growth factor (TGF) B to suppress general antitumor immune responses. Meanwhile, TAMs promote tumor neo-angiogenesis by the secretion of pro-angiogenic factors and define the invasive microenvironment to facilitate tumor metastasis and dissemination. For these reasons, reducing the pool of anti-inflammatory TAMs has been considered as a relevant approach to anti-cancer therapy.

It has been previously reported that the NLR family member NLRP3 and the purinergic receptor P2Y2 physically interact (Paoletti et al., Cell Rep (2019)), control macrophage functions (such as cytokine secretion, functional reprogramming and migration) and constitutes an innate immune checkpoint that acts on macrophages (WO 2016/185026). In this prior work, it was proposed to use P2Y2R inhibitors in order to enhance the pool of pro-inflammatory macrophages and treat viral infections. It was furthermore shown that P2Y2R agonists can reduce the macrophage pro-inflammatory polarization, reduce the secretion of inflammatory cytokines, increase the anti-inflammatory macrophages pool and therefore be useful in patients suffering from auto-immune diseases or inflammatory diseases.

The present inventors have now observed that P2Y2R agonists can surprisingly inhibit the hyper-inflammation that is detected in patients with COVID-19 and can also impair the replication of the virus. More precisely, they revealed that P2Y2 receptor agonists (such as UTP, Diquafosol and Denufosol) can reduce macrophage pro-inflammatory reprogramming, NLRP3 inflammasome activation and subsequent IL-1β secretion in response to IFNγ or LPS+ATP stimulation and can impair the replication and the cytopathogenic effects of SARS-CoV-2. They therefore propose to reprogram macrophage pro-inflammatory phenotype into anti-inflammatory phenotype through the modulation of NLRP3-P2Y2 immune checkpoint as a therapeutic option for treating patients with COVID-19.

The present inventors furthermore studied the effects of other modulators of purinergic receptors on the macrophage populations and on the inflammasome activation in COVID suffering patients.

They thereby observed that P2X receptor antagonists (such as pyridoxal phosphate-6-azophenyl-2',4'-disulfonic acid (PPDAS)) can repress SARS-CoV2 viral replication, whereas the P2X7 receptor agonist 2',3'-O-(4-benzoyl-benzoyl)ATP enhanced the viral replication. They therefore propose to use P2X receptor antagonists as a therapeutic option for treating patients with COVID-19.

Altogether, these results demonstrate that purinergic receptors and their ligands (the extracellular nucleotides) play a central role during SARS-CoV-2 infection. The present application provides the first evidence that therapeutic manipulation of these receptors represents a good opportunity for the treatment of COVID-19.

### Modulators of the invention

In a first aspect, the present invention therefore proposes to modulate purinergic receptors and/or the related immune checkpoint (in particular the NLRP3-P2Y2 immune checkpoint) for treating subjects suffering from Acute Respiratory Disease Syndrome (ARDS). This modulation can be obtained by means of various "modulators", which are explained below.

In a particular embodiment, said modulator can be a modulator of purinergic receptors. This modulation can be either direct or indirect. Direct modulation can be mediated by using agonists or antagonists of said receptors. Indirect modulation can be obtained by modifying the availability or the level of the ligands of these receptors, or by any means enabling to enhance or reduce the biological activity of these receptors indirectly.

**Purinergic receptors**, also known as **purinoceptors**, are a family of plasma membrane molecules that are found in almost all mammalian tissues. More specifically, they are involved in several cellular functions, including proliferation and migration of neural stem cells, vascular reactivity, apoptosis and cytokine secretion. The term *purinergic receptor* was originally introduced to illustrate specific classes of membrane receptors that mediate relaxation of gut smooth muscle as a response to the release of ATP (P2 receptors) or adenosine (P1 receptors). P2 receptors have further been divided into five subclasses: P2X, P2Y, P2Z, P2U, and P2T. To distinguish P2 receptors further, the subclasses have been divided into families of metabotropic (P2Y, P2U, and P2T) and ionotropic receptors (P2X and P2Z).

In a preferred embodiment, the modulator of the purinergic receptor is a direct modulator which is selected from: an agonist of a purinergic P2Y receptor and an antagonist of a purinergic P2X receptor.

P2Y receptors are a family of purinergic G protein-coupled receptors. They are activated by ATP, ADP, UTP, UDP and UDP-glucose. They are known to be widely distributed in the brain, heart, kidneys, and adipose tissue. To date, 8 P2Y receptors have been cloned in humans: P2Y1, P2Y2, P2Y4, P2Y6, P2Y11, P2Y12, P2Y13 and P2Y14. They display large-scale structural domains typical of GPCRs, consisting of seven hydrophobic transmembrane helices connected by three short extracellular loops and three variably sized intracellular loops; an extracellular N-terminus; and an intracellular C-terminus. The extracellular regions interact with the receptor ligands, while the intracellular regions activate the G protein, control receptor internalization, and mediate dimerization. Similar to other GPCRs, P2Y receptors can form both homodimers and heterodimers. These dimeric forms may vary significantly in their biochemical and pharmacological properties from the monomeric receptor. In addition to the structural domains typical of all GPCRs, some structural elements are common across **P2Y receptor subtypes.** All P2Y receptors contain four extracellular cysteine residues which can form two disulfide bridges, one between the N-terminus domain and the proximal extracellular loop and another between the two remaining extracellular loops. These disulfide bonds have been shown to be involved in ligand binding and signal transduction. In addition, several polar residues found within the transmembrane helices are highly conserved across both species and receptor subtypes. Mutational analysis has suggested that these residues are integral to the ligand-binding mechanism of P2Y receptors. Outside of these conserved regions, the P2Y receptor family exhibits unusually high diversity in primary structure, with P2Y1 sharing only 19% of its primary structure with P2Y12. Despite this, the individual P2Y subtypes are highly conserved across species, with human and mouse P2Y receptors sharing 95% of amino acids.

P2Y receptors respond either positively or negatively to the presence of nucleotides in extracellular solution. Nucleotides may be divided into two categories: purines and pyrimidines. Some P2Y receptor species may respond to only purines, only pyrimidines, or both; for example, P2Y1 respond only to purines, P2Y4 respond only to pyrimidine, P2Y14 is activated only by UDP-glucose, and P2Y2 is activated by both purines and pyrimidines triphosphate (Tulapurkar ME, 2005).

The activity of P2Y receptors is linked to a signal cascade originating in regulation of the flow of Ca2+ and K+ ions by the receptor's interactions with G proteins, modulating access to Ca2+ and K+ channels. Voltage-independent Ca2+ channels allow for the free flow of Ca2+ ions from the cell activated by P2Y receptors (Van Kolen K, 2006).

By contrast, **P2X receptors** are ligand-gated ion channels. These ligand-gated ion channels are nonselective cation channels responsible for mediating excitatory postsynaptic responses, similar to nicotinic and ionotropic glutamate receptors. P2X receptors are distinct from the rest of the widely known ligand-gated ion channels, as the genetic encoding of these particular channels indicates the presence of only two transmembrane domains within the channels. These receptors are greatly distributed in neurons and glial cells throughout the central and peripheral nervous systems. P2X receptors mediate a large variety of responses including fast transmission at central synapses, contraction of smooth muscle cells, platelet aggregation, macrophage activation, and apoptosis (North RA et al, Physiological Reviews. 82(4):1013-67).

P2X receptors are expressed in cells from a wide variety of animal tissues. P2X receptors are able to initiate contraction in cells of the heart muscle, skeletal muscle, and various smooth muscle tissues, including that of the vasculature, vas deferens and urinary bladder. P2X receptors are also expressed on leukocytes, including lymphocytes and macrophages, and are present on blood platelets. There is some degree of subtype specificity as to which P2X receptor subtypes are expressed on specific cell types, with P2X1 receptors being particularly prominent in smooth muscle cells, and P2X2 being widespread throughout the autonomic nervous system. However, such trends are very general and there is considerable overlap in subunit distribution, with most cell types expressing more than one subunits. For example, P2X2 and P2X3 subunits are commonly found co-expressed in sensory neurons, where they often co-assemble into functional P2X2/3 receptors.

Generally speaking, most subunits can form functional homomeric or heteromeric receptors. Receptor nomenclature dictates that naming is determined by the constituent subunits; e.g. a homomeric P2X receptor made up of only P2X1 subunits is called a P2X1 receptor, and a heteromeric receptor containing P2X2 and P2X3 subunits is called a P2X2/3 receptor. The general consensus is that P2X6 cannot form a functional homomeric receptor and that P2X7 cannot form a functional heteromeric receptor. Topologically, they resemble the epithelial Na+ channel proteins in possessing (a) N- and C-termini localized intracellularly, (b) two putative transmembrane segments, (c) a large extracellular loop domain, and (d) many conserved extracellular cysteyl residues. P2X receptor channels transport small monovalent cations, although some also transport Ca²⁺. Evidence has strongly indicated that the functional P2X receptor protein is a trimer, with the three peptide subunits arranged around an ion-permeable channel pore (Kawate T, 2009). These findings indicate that the second transmembrane domain of each subunit lines the ion-conducting pore and is therefore responsible for channel gating.

The P2X receptors open in response to the binding of extracellular adenosine 5' triphosphate (ATP). Three ATP molecules are thought to be required to activate a P2X receptor, suggesting that ATP needs to bind to each of the three subunits in order to open the channel pore, though recent evidence suggests that ATP binds at the three subunit interfaces. Once ATP binds to the extracellular loop of the P2X receptor, it evokes a conformational change in the structure of the ion channel that results in the opening of the ion-permeable pore. The most commonly accepted theory of channel opening involves the rotation and separation of the second transmembrane domain (TM) helices, allowing cations such as Na⁺ and Ca²⁺ to access the ion-conducting pore through three lateral fenestrations above the TM domains. The entry of cations leads to the depolarization of the cell membrane and the activation of various Ca²⁺⁻sensitive intracellular processes.

As used herein, the term "**P2Y2 receptor**" has its general meaning in the art and refers to the P2Y purinoreceptor 2 also known as P2RY2, HP2U, P2RU1, P2U, P2U1, P2UR, P2Y2, P2Y2R, and purinergic receptor P2Y2. This receptor protein of 377 amino acids is a G-protein-coupled receptor with seven transmembrane-spanning domains. It is referenced in public available bases as NP_002555 (SEQ ID NO:1). Said receptor protein is encoded by the *P2RY2* gene in humans. Three human transcript variants encode the same 377 amino acid protein sequence: NM_002564, NM_176071, NM_176072.

As used herein, the term "**P2X7 receptor**" has its general meaning in the art and refers to the P2X purinoreceptor 7 known depicted as NP_002553 (SEQ ID NO:2). It is a 595-amino acid polypeptide with two membrane-spanning domains. Said receptor is encoded by the P2X7 gene in humans, for example by the mRNA NM_002562.

In another particular embodiment of the invention, the modulator of the invention is able to modulate the NLRP3-P2Y2 immune checkpoint. In this case, said modulator is for example able to impair the activity of the NLRP3 inflammasome.

The NLRP3 inflammasome, so-called because the NLRP3 protein in the complex belongs to the family of nucleotide-binding and oligomerization domain-like receptors (NLRs), is also known as "pyrin domain-containing protein 3". NLRP3 assembles a multimeric inflammasome complex serving as an activation platform for caspase-1 that controls processing and release of cytosolic inflammatory factors and cytokines including IL-1B. Inflammasome assembly is tightly controlled and requires coordinated NLRP3 priming, through cytokine or other pattern recognition receptors, followed by activation by cellular stress. The present inventors demonstrated that it is involved in the pro-inflammatory response associated with virus-induced ARDS, notably due to SARS-CoV2.

As used herein, the term "**NLRP3**" has its general meaning in the art and refers to the "NACHT, LRR and PYD domains-containing protein 3". An exemplary human amino acid sequence is represented by NP_004886 (isoform a of 1036 amino acids).

Several NLRP3 inflammasome inhibitors have been described, some of which show promise in the clinic (see below and in Shao B-Z et al, 2015).

To reduce the inflammasome activity, it is also possible to use compounds that act as antagonists of NRLP3. These compounds are for example Troxerutin, Tranilast or Glyburide.

It is possible to detect the inflammasome activity by common molecular means (such as CaspaseGlo® of Promega, detection of IL-1b secretion using ELISA or Western blot or NLRP3 inflammasome formation using Fluorescence Resonance Energy Transfer (FRET) Assay).

### Compounds of the invention

As used herein, the term "**P2Y receptor agonist**" refers to any compound that enhances the biological activity of at least one of the P2Y receptors as defined above (P2Y1, P2Y2, P2Y4, P2Y6, P2Y11, P2Y12, P2Y13 and P2Y14).

In a preferred embodiment, said P2Y receptor agonist of the invention is able to enhance the activity of the P2Y2 receptor. In the presence of this agonist, the P2Y2 receptor will perform its biochemical or its cellular function with enhanced efficiency. For example, such an agonist can act by making the receptor more accessible to its natural ligand (e.g. ATP) so that its normal biological activity is enhanced. The agonistic activity of compounds towards the P2Y2 receptors may be determined using various methods well known in the art. For example, the assay can be performed with P2Y2 receptor expressed on the surface of cells. A typical assay for determining the agonistic activities of a compound on P2Y2 receptor is described in P. Hillmann, et al.,2009 and in Van Poecke S. et al, 2012.

By "agonist", it is herein meant either a small chemical molecule or a larger protein (such as antibody) that interact physically with the target receptor so as to enhance its biological activity, e.g., by rendering the receptor more accessible to its ligand.

Chemical P2Y2 receptor agonists are well known in the art and include those described in US 5,789,391, US 5,837,861, and US 2002/0082417. Suitable chemical P2Y2R agonists typically include INS-37217 [P(1)-(uridine 5')-P(4)-(2'-deoxycytidine 5')tetraphosphate, tetrasodium salt], uridine 5'triphosphate, diquafosol tetrasodium, and the like. In some embodiments, P2Y2 receptor agonists are selected from the compounds described in WO 2008/060632, which is incorporated herein by reference. It is also possible to use activating antibodies. With this respect, monoclonal antibodies can be produced and screened for their capacity to enhance the activity of the P2Y2 receptor.

In a more preferred embodiment, the P2Y receptor agonist of the invention is a small chemical compound selected in the group consisting of: MRS2698, Uridine triphosphate (UTP), 4-thio-UTP, 2-thioUTP, Diquafosol, PSB1114, ATP, Denufosol, Ap4A, UTPγS, 5BrUTP and MRS2768 and any pharmaceutically acceptable salt thereof (Kenneth A. et al, 2012; Kenneth A. et al, 2009).

As used herein, the term "**P2X receptor antagonist**" refers to any compound that impairs or blocks or reduces the biological activity of at least one of the P2X receptors as defined above (P2X1, P2X2, P2X3, P2X4, P2X5, P2X6, P2X7). The antagonistic activity of compounds towards the P2X receptors may be determined using various methods well known in the art. For example, the agents may be tested for their capacity to block the interaction of P2X receptor with its natural ligand receptor (e.g. periodate-oxidized ATP), or to reduce the biological activity of the P2X receptor without impairing the binding of the ligand.

By "antagonist", it is herein meant either a small chemical molecule that can directly interact with the target receptor, or polypeptides (such as antibodies or aptamers) that can block the interaction between the target receptor and its ligand. More generally, it can also encompass gene expression inhibitors (siRNAs, ribozymes, etc) that can inhibit the production of the protein in target cells.

Chemical P2X receptor antagonists are for example NF279 (P2X1 antagonist), NF449 (P2X1 antagonist), Suramin (P2X1 and P2X5 antagonist), TNP-ATP (P2X1 and P2X4 antagonist), Ip5I (P2X1 antagonist) and NF023 (P2X1 antagonist), NF778 (P2X2 antagonist), NF770 (P2X2 antagonist), A317491 (P2X3 antagonist), gefapixant (P2X3 antagonist), PSB-12062 (P2X4 antagonist), 5-BDBD (P2X4 antagonist) and any pharmaceutically acceptable salt thereof (North R.A. and Jarvis M.F., 2013). It is also possible to use antibodies / aptamers that target the binding site of P2X receptor in order to impair the binding of its ligand.

In a preferred embodiment, the P2X receptor antagonist of the invention is able to impair the activity of the P2X7 receptor.

In a preferred embodiment, the P2X receptor antagonist of the invention is able to impair the activity of the P2X7 receptor and is chosen in the group consisting of: JNJ-47965567 (Bhattacharya A, et al.. (2013) Br. J. Pharmacol., 170 (3): 624-40), Compound 16i (Homerin G, et al. (2019) J. Med. Chem.), AZ10606120 (Guile SD, et al (2009) J. Med. Chem., 52 (10): 3123-41; Michel AD, et al. (2008) Br. J. Pharmacol., 153 (4): 737-50), AZD9056 (McInnes IB, et al (2014) Clin. Exp. Rheumatol., 32 (6): 878-82), GSK1482160 (Abdi MH, et al.. (2010) Bioorg. Med. Chem. Lett., 20 (17): 5080-4 ; Homerin G, et al.. (2019) J. Med. Chem.*),* A438079 (Donnelly-Roberts DL, Jarvis MF. (2007) Br. J. Pharmacol., 151 (5): 571-9 ; Khalafalla MG, et al. (2017) J. Biol. Chem., 292 (40): 16626-16637), BBG (Marta R S Carmoet al., Neuropharmacology 2014 Jun;81:142-52), KN62 (Gargett CE, Wiley JS. (1997) Br. J. Pharmacol., 120 (8): 1483-90),oxATP (Owe, p.n. & Beechey, r.b. (1982). Biochemistry, 21, 4073-4082), A74003 [N-(1-{[(cyanoimino)(5-quinolinylamino)methyl]amino}-2,2-dimethylpropyl)-2-(3,4-dimethoxyphenyl)acetamide] (Honore et al (2006) J.Pharmacol.Exp.Ther. 319 1376), AACBA [N-(adamantan-1-ylmethyl)-5-[(3R-amino-pyrrolidin-1-yl)methyl]-2-chloro-benzamide] (Daniel C Broom, et al. J Pharmacol Exp Ther 2008 Dec;327(3):620-33) and CE-224,535 (or PF-04905428) (Duplantier AJ, et al.. (2011) Bioorg. Med. Chem. Lett., 21 (12): 3708-11) and any pharmaceutically acceptable salt thereof.

It is also possible to use antibodies /aptamers that target the binding site of P2X7 receptor in order to impair the binding of its ligand. In this embodiment, the inhibitor of P2X receptor may consist in an antibody (this term including antibody fragment). In particular, it can be an antibody directed against the P2X receptor in such a way that said antibody impairs the activation of said receptor. Antibodies can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies can be prepared and isolated using any technique that provides for the production of antibody molecules by continuous cell lines in culture. Techniques for production and isolation include but are not limited to the hybridoma technique; the human B-cell hybridoma technique; and the EBV-hybridoma technique. Alternatively, techniques described for the production of single chain antibodies (see, e.g., U.S. Pat. No. 4,946,778) can be adapted to produce anti-P2X receptor, single chain antibodies. The inhibitor of P2X receptor activity of the invention also include anti-P2X receptor antibody fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to the receptor or channel. Humanized antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397). In view of these information, the skilled in the art can easily generate and select antibodies blocking the P2X receptor.

The inhibitor of P2X can also be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods. In view of these information, the skilled in the art can easily generate and select aptamers blocking the P2X receptor.

In the context of the invention, it is also possible to use inhibitors of P2X receptor expression, that will efficiently reduce or abolish the activity of the P2X receptor. Such inhibitor can be used so that (i) the transcription of the gene encoding P2X receptor is lowered, i.e. the level of its mRNA is lowered or (ii) the translation of the mRNA encoding P2X receptor is lowered.

The P2X modulator of the invention can also inhibit the P2X receptor gene expression. It is for example a natural or synthetic compound that has a biological effect to inhibit or significantly reduce the expression of a P2X gene, e.g., of the *P2X7R* gene. Inhibitors of gene expression for use in the present invention may be based on anti-sense oligonucleotide constructs. Anti-sense oligonucleotides, including anti-sense RNA molecules and anti-sense DNA molecules, would act to directly block the translation of the mRNA by binding thereto and thus preventing protein translation or increasing mRNA degradation, thus decreasing the level of the protein (e.g. P2X receptor), and thus activity, in a cell. For example, antisense oligonucleotides of at least about 15 bases and complementary to unique regions of the mRNA transcript sequence encoding the targeted protein (e.g. P2X receptor) can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). Small inhibitory RNAs (siRNAs) can also function as inhibitors of gene expression for use in the present invention. Gene expression can be reduced by contacting a subject or cell with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone. The antisense oligonucleotides siRNAs and ribozymes of the invention may be delivered *in vivo* alone or in association with a vector. Preferred vectors are defined below.

It is also possible to modulate indirectly the activity of purinergic receptors by modifying the availability and/or the quantity of their ligand (e.g., ATP) in the surroundings of the macrophage cells.

In another embodiment, the modulator used in the invention is therefore able to reduce the level of circulating extracellular nucleotides such as ATP. To do so, it is possible to use, e.g., the human recombinant apyrase AZD3366 (ATP102) (Douglas Moeckel, et al., Sci Transl Med. 2014 Aug 6;6(248):248ra105).

In the examples below, the inventors have shown that the use of P2Y2 agonists trigger the inactivation of the NLRP3 inflammasome in activated macrophages. It is therefore also possible to partially mimic the effect of the P2Y2 agonists by using NLRP3 inflammasome inhibitors such as MCC950 (Coll RC, et al. Nat Med. 2015 Mar;21 (3):248-55) or beta-Hydroxybutyrate (Yun-Hee Youm, et al. Nat Med. 2015 Mar;21 (3):263-9), or NLR3P antagonists such as Troxerutin (Chunhui Sun, et al. Journal of Functional Foods. Volume 26, October 2016, Pages 598-609), Tranilast (Yi Huang, et al. EMBO Mol Med 2018 Apr;10(4):e8689) and Glyburide (Mohamed Lamkanfi, et al. J Cell Biol 2009 Oct 5; 187(1):61-70).

It is also possible to use, as for P2X7R, any inhibitor that affect the expression level of NLRP3 (siRNAs, ribozymes, etc) or any antibody/aptamer that block the activity of the NLRP3 protein.

The term "P2Y2R modulator" therefore also encompasses, in the context of the invention, NLRP3 antagonists such as Troxerutin, Tranilast and Glyburide, and NLR3 inflammasome inhibitors such as MCC950 or beta-Hydroxybutyrate.

By "modulators of the invention" or "compounds of the invention", it is therefore herein encompassed:
- A modulator of purinergic receptors, preferably an agonist of a purinergic P2Y receptor or an antagonist of a purinergic P2X receptor, more preferably an agonist of the purinergic P2Y2 receptor or an antagonist of the purinergic P2X7 receptor, as defined above,
- A modulator that impair the activity of the NRLP3 inflammasome, e.g., an antagonist of NLR3P or an NLRP3 inflammasome inhibitor, as defined above, and
- A modulator that reduce the level of extracellular nucleotides such as ATP, as defined above.

### Therapeutic uses of these modulators

The examples below suggest that the modulation of NLRP3-P2Y2 immune checkpoint and other purinergic receptor signaling pathways represent an innovative therapeutic strategy to avoid SARS-CoV-2-induced Acute Respiratory Distress Syndrome (ARDS).

The present invention therefore relates on the use of the modulators of the invention for treating subjects suffering from an acute respiratory distress syndrome ARDS.

The present invention also encompasses the use of any of the compound of the invention for manufacturing a pharmaceutical composition intended to treat subjects suffering from ARSD. In these pharmaceutical compositions, the compounds of the present invention (e.g. P2Y2 receptor agonist or the inhibitor of P2X receptor activity) are typically combined with pharmaceutically acceptable excipients or sustained-release matrices (such as biodegradable polymers). The present invention also relates to treatment methods comprising the step of administering a therapeutically effective amount of the modulators / compounds of the invention to subjects in need thereof.

**Acute respiratory distress syndrome** (ARDS) is a type of respiratory failure characterized by rapid onset of widespread inflammation in the lungs (Fan E. et al, JAMA 2018). Symptoms include shortness of breath, rapid breathing, and bluish skin coloration. The underlying mechanism involves diffuse injury to cells which form the barrier of the microscopic air sacs of the lungs, surfactant dysfunction, activation of the immune system, and dysfunction of the body's regulation of blood clotting (Fanelli V. et al, Annals of the American Thoracic Society, 2015). ARDS impairs the lungs' ability to exchange oxygen and carbon dioxide. Adult diagnosis is based on a PaO₂/FiO₂ ratio (ratio of partial pressure arterial oxygen and fraction of inspired oxygen) of less than 300 mm Hg despite a positive end-expiratory pressure (PEEP) of more than 5 cm H₂O. The primary treatment involves mechanical ventilation together with treatments directed at the underlying cause. Ventilation strategies include using low volumes and low pressures. If oxygenation remains insufficient, lung recruitment agents and neuromuscular blockers may be used. If these are insufficient, extracorporeal membrane oxygenation (ECMO) may be an option. The syndrome is associated with a death rate between 35 and 50% (Fan E. et al, JAMA 2018).

The acute respiratory distress syndrome (ARDS) can be induced by various causes, as shown in Table 1 below, which is an abbreviated list of the common causes of ARDS.

**Table 1. Etiology of ARDS: acute respiratory distress syndrome; DIC: disseminated intravascular coagulation; HSCT: hematopoietic stem cell transplant; AEP: acute eosinophilic pneumonia; COP: cryptogenic organizing pneumonia; DAD: diffuse alveolar.**

| **Etiology** | **Clinical features** | **Diagnostic tests** |
|---|---|---|
| Sepsis | Fever hypotension, leukocytosis, lactic acidosis, infectious source | Appropriate clinical context and positive cultures |
| Aspiration pneumonitis | Witnessed or risk for aspiration, food, lipid laden macrophages, airway erythema on bronchoscopy | Presumptive diagnosis with negative cultures |
| Infectious pneumonia (including mycobacterial, viral, fungal, parasitic) | Productive cough, pleuritic pain, fever, leukocytosis, lobar consolidation or bilateral infiltrates in an immunosuppressed patient | Appropriate clinical context and positive respiratory cultures |
| Severe trauma and/or multiple fractures | History of trauma or fractures within the last week | Diagnosis is apparent |
| Pulmonary contusion | History of chest trauma (blunt or penetrating), chest pain | Presumptive diagnosis in the correct clinical context, negative cultures |
| Burns and smoke inhalation | Exposure to fire or smoke, cough, dyspnea, DIC, particulate matter on bronchoscopy, surface burns | Presumptive diagnosis in the correct clinical context, negative cultures |
| Transfusion related acute lung injury and massive transfusions | History of transfusion, dyspnea during or shortly after transfusion | Diagnosis of exclusion |
| HSCT | History of HSCT | Diagnosis of exclusion |
| Pancreatitis | Abdominal pain, vomiting, risk actors (eg, gallstones, alcohol, viral infection) | Elevated amylase and lipase, with or without abnormal imaging |
| Inhalation injures other than smoke (eg, near drowning, gases) | History of inhalation exposure (eg, chlorine gas) | Diagnosis of exclusion |
| Thoracic surgery (eg, post-cardiopulmonary bypass) or other major surgery | History of surgery, intraoperative ventilation, intraoperative transfusion | Diagnosis of exclusion |
| Drugs (chemotherapeutic agents, amiodarone, radiation) | New drugs or radiation exposure on history, lymphocytosis on lavage, lavage may have suggestive features of amiodarone toxicity ("foamy macrophages") but is nonspecific | Diagnosis of exclusion, lung biopsy occasionally helpful |

It is possible to use the modulators of the invention to treat ARDS whatever its etiology is. In particular, it is possible to use the modulators of the invention to treat ARDS caused by sepsis, pneumonia, pancreatitis, surgery, radiation or chemotherapeutic drugs, etc.

ARDS is a clinical diagnosis of exclusion: it can only be diagnosed once cardiogenic pulmonary edema and alternative causes of acute hypoxemic respiratory failure and bilateral infiltrates have been excluded. The Berlin Definition of ARDS requires that all of the following criteria be present for diagnosis:
- Respiratory symptoms must have begun within one week of a known clinical insult, or the patient must have new or worsening symptoms during the past week.
- Bilateral opacities must be present on a chest radiograph or computed tomographic (CT) scan. These opacities must not be fully explained by pleural effusions, lobar collapse, lung collapse, or pulmonary nodules.
- The patient's respiratory failure must not be fully explained by cardiac failure or fluid overload. An objective assessment (e.g., echocardiography) to exclude hydrostatic pulmonary edema is required if no risk factors for ARDS are present.
- A moderate to severe impairment of oxygenation must be present, as defined by the ratio of arterial oxygen tension to fraction of inspired oxygen (PaO₂/FiO₂).

The severity of the hypoxemia defines the severity of the ARDS:
- Mild ARDS - The PaO₂/FiO₂ is >200 mmHg, but ≤300 mmHg, on ventilator settings that include positive end-expiratory pressure (PEEP) or continuous positive airway pressure (CPAP) ≥5 cm H₂O.
- Moderate ARDS - The PaO₂/FiO₂ is > 100 mmHg, but ≤200 mmHg, on ventilator settings that include PEEP ≥5 cm H₂O.
- Severe ARDS - The PaO₂/FiO₂ is ≤100 mmHg on ventilator settings that include PEEP ≥5 cm H₂O.

Determining the PaO₂/FiO₂ requires arterial blood gas (ABG) analysis. To calculate the PaO₂/FiO₂ ratio, the PaO₂ is measured in mmHg and the FiO₂ is expressed as a decimal between 0.21 and 1. As an example, if a patient has a PaO₂ of 60 mmHg while receiving 80 percent oxygen, then the PaO₂/FiO₂ ratio is 75 mmHg (ie, 60 mmHg/0.8). For patients in whom an ABG cannot be obtained, the ratio of oxyhemoglobin saturation measured by pulse oximetry (SpO2) to FiO₂ may be an appropriate substitute.

In addition, ARDS can be induced by viruses. Two virus types have been involved in the aetiology of this disease: respiratory viruses that cause community-acquired viral pneumonia and Herpesviridae that cause nosocomial viral pneumonia (Luyt et al, Presse Med 2011). Among the respiratory viruses that can affect the lung and cause ARDS, pandemic viruses head the list, with influenza viruses H5N1 and H1N1 2009 being recently identified. Other viruses can cause severe ARDS. Notably, novel coronaviruses have been responsible for the severe acute respiratory syndrome outbreaks in 2003 and in 2019.

In a particular embodiment, the present invention relates on the use of the modulators of the invention for treating subjects suffering from a virus-induced acute respiratory distress syndrome ARDS.

In a preferred embodiment, the modulators of the invention are thus administered to subjects suffering from an ARDS caused by an influenza virus (such as H1N1 or H5N1), a respiratory virus, or an herpesvirus. All these viruses have indeed been shown to cause ARDS in infected humans (Luyt et al, Presse Med 2011).

The term "herpesvirus" (or "Herpesviridae") herein designates any herpesvirus that has been shown to induce an ARDS in an animal. It can be for example the Herpes simplex virus (HSV) or the Cytomegalovirus (CMV) (Luyt et al, Presse Med 2011).

The term "respiratory virus" herein encompasses parainfluenza viruses, adenoviruses, respiratory syncytial viruses, coronaviruses and the metapneumovirus.

Coronaviruses are enveloped viruses with a helically symmetrical capsid. They have a single-stranded, positive-sense RNA genome and are capable of infecting cells in birds and mammals. The morphology of the virions is typical, with a halo of protein protuberances ('Spike') which gave them their name of 'crown virus'. Four genera have been identified: alphacoronavirus, betacoronavirus, gammacoronavirus, deltacoronavirus. They can all infect humans.

In one embodiment, the modulators of the invention are administered to subjects that have been infected by at least one coronavirus.

In a particular embodiment, the modulators of the invention are administered to subjects that have been infected by at least one betacoronavirus.

The *Betacoronavirus* genus (β-CoVs or Beta-CoVs), comprising virus infecting animals and/or humans, is subdivided into four lineages designated as A, B, C and D: Lineage A also designated as subgenus *Embecovirus* includes HCoV-OC43 and HCoV-HKU1, virus able to infect various species; Lineage B also designated as subgenus *Sarbecovirus* includes SARS-CoV-1, SARS-CoV-2, and Bat SL-CoV-WIV1; Lineage C also designated as subgenus *Merbecovirus* includes Tylonycteris bat coronavirus HKU4 (BtCoV-HKU4), Pipistrellus bat coronavirus HKU5 (BtCoV-HKU5), and MERS-CoV, able to infect notably camels and humans; Lineage D also designated as subgenus *Nobecovirus* includes Rousettus bat coronavirus HKU9 (BtCoV-HKU9).

As used herein, the term "**betacoronavirus**" designates any virus belonging to the *Betacoronavirus* genus (β-CoVs or Beta-CoVs) within the *Coronaviridae* family, in particular any *betacoronavirus* belonging to one of the four lineages designated as A, B, C and D. It designates a betacoronavirus infecting animals (preferably a mammal) and/or humans. In particular, this designation includes the betacoronaviruses infecting human organisms selected from the group consisting of OC43, HKU1, SARS-CoV-1, SARS-CoV-2 and MERS-CoV.

The Betacoronaviruses of the greatest clinical importance concerning humans are:
- OC43 and HKU1 of the A lineage,
- SARS-CoV-1 and SARS-CoV-2 of the B lineage, and
- MERS-CoV of the C lineage.

In a more preferred embodiment, the modulators of the invention are administered to subjects that have been infected by a betacoronavirus (such as OC43, HKU1, MERS-CoV, SARS-CoV-1 and SARS-CoV-2). In this preferred embodiment, the subjects treated by the invention are said to suffer from a COVID disease. As used herein, the terms "**COVID disease**" or "**COVID**" or "**betacoronavirus disease**" mean the disease linked to (associated with) the infection with at least one betacoronavirus, as listed above.

In an even more preferred embodiment, the modulators of the invention are administered to subjects that have been infected by the SARS-CoV-2 virus. "**SARS-CoV-2**" herein refers to Severe Acute Respiratory Syndrome Coronavirus 2. SARS-CoV-2 belongs to the species *Coronavirus,* in the genus *Betacoronavirus* and family *Coronaviridae.* In this embodiment, the subjects treated by the invention are said to suffer from a COVID 19 disease. As used herein, the terms "**COVID-19 disease**" or "**COVID-19**" or "**coronavirus disease 19**" indeed mean the disease linked to (or associated with) the infection with (at least) the SARS-CoV-2 (Severe Acute Respiratory Syndrome Coronavirus 2).

Viral diseases can manifest several forms, depending on the severity of the symptoms/signs. They can be asymptomatic in some people. They can induce a simple fever accompanied by cough in others. The early COVID symptoms (such as COVID-19) comprise: dry cough, muscle pain, headache, fever, fatigue, loss of taste or smell. They can ultimately cause acute respiratory distress and death.

In particular, for the betacoronavirus disease (such as COVID-19), the following forms are usually observed:
- an "**asymptomatic form**" wherein the subject is a carrier of at least on betacoronavirus but shows no symptom.
- a "**mild COVID form**" wherein the subject shows the first symptoms (or signs) of a COVID disease, as listed above.. Mild COVID symptoms (such as COVID-19) comprise e.g., mild dry cough, mild muscle pain, mild headache, mild fever, mild fatigue, loss of taste or smell.
- a "**strong COVID form**" wherein the subject shows strong respiratory symptoms such as difficulty breathing, lack of oxygen; other stronger symptoms such as fever, dry cough, aches and pains, nasal congestion, strong headache, conjunctivitis, sore throat, skin rash, discoloration of fingers or feet, or any combination thereof; as well as a deterioration of the general state of health with frequent diarrhoea, but also liver or urinary disorders, dizziness or neuromuscular problems; some of these symptoms may require hospitalisation. Most patients have an abnormal chest X-ray or CT scan within the first few days of illness, even in the absence of respiratory signs.
- A "**severe COVID form**" or "**critical COVID form**" or "**aggressive COVID form**" wherein the subject has life-threatening symptoms (or signs) of COVID (e.g. COVID-19), comprising at least one selected from (but not limited to) : respiratory distress, lung disorders, liver disorders, kidney disorders, neuromuscular disorders, brain disorders, etc, that requires hospitalisation and/or intensive care (in intensive care units (ICU)).

More generally, it is possible to use the modulators of the invention to treat inflammatory diseases that are associated to an accumulation of pro-inflammatory macrophages and/or associated with an over activation of the NRLP3 inflammasome. These inflammatory diseases are for example the cryopyrin associated periodic syndromes (Agostini et al., 2004), rheumatoid arthritis (Van de Walle et al., 2014), obesity (Vandanmagsar et al., 2011) or Alzheimer's disease (Halle et al., 2008).

As used herein, a "**subject**" or an "**individual**" is an animal, preferably a mammal, including, but not limited to, human, dog, cat, cattle, goat, pig, swine, sheep and monkey. More preferably, the subject is a human subject. A human subject can be known as a patient.

As used herein, "**subject in need**" refers to an animal, preferably a mammal, more preferably a human, that suffer from, or is susceptible to suffer from any aetiology of ARDS, as explained above.

This subject may suffer three different stages of ARDS :
- Early exudative stage (DAD) - The early exudative stage during the first 7 to 10 days is characterized by DAD. DAD is a nonspecific reaction to lung injury from a variety of causes. It is characterized by interstitial edema, acute and chronic inflammation, type II cell hyperplasia, and hyaline membrane formation.
- Fibroproliferative stage - After approximately 7 to 10 days, a proliferative stage develops, characterized by resolution of pulmonary edema, proliferation of type II alveolar cells, squamous metaplasia, interstitial infiltration by myofibroblasts, and early deposition of collagen. It is unknown how long this phase lasts but is probably in the realm of two to three weeks.
- Fibrotic stage - Some patients progress to a fibrotic stage, characterized by obliteration of normal lung architecture, fibrosis, and cyst formation. The degree of fibrosis ranges from minimal to severe.

Preferably, the subject in need to be treated by the modulators of the invention is in an early exudative or in a fibroproliferative stage of ARDS.

More preferably, said subject in need suffers from a virus-induced ARDS, more preferably from a betacoronavirus-induced ARDS, even more preferably, from SARS-COV2-induced ARDS.

In this latter case, said subject is a "**COVID suffering subject**" i.e., an animal, preferably a mammal, more preferably a human, that suffers from COVID and/or has been diagnosed with COVID and/or is infected with at least one betacoronavirus and/or suffers from at least one betacoronavirus infection. Even more preferably, said subject is a "**COVID-19 suffering subject**", i.e., an animal, preferably a mammal, more preferably a human, that suffers from COVID-19 and/or has been diagnosed with COVID-19 and/or is infected with SARS-CoV2 and/or suffers from a SARS-CoV2 infection.

Diagnosis of a viral infection can be done by any known molecular means enabling to detect the presence of a virus in a biological sample (e.g., blood) of the subject. A number of diagnostic tools have been generated in the recent months to detect the SARS COV 2 virus specifically.

The tested subject is preferably suffering from a mild COVID form, displaying at least one of the symptom defined above (mild dry cough, mild muscle pain, mild headache, mild fever, mild fatigue, loss of taste or smell) and being diagnosed with COVID. He/she can also suffer from an asymptomatic form or from a strong COVID form, as defined above. In the first case, the treatment of the invention will permit to prevent the occurrence of the symptoms of the infection (mild dry cough, mild muscle pain, mild headache, mild fever, mild fatigue, loss of taste or smell). In the second case, the treatment of the invention will permit to alleviate the severe symptoms and diminish the risk of death for the patient.

The subjects to be treated can suffer only from said viral infection. They can also suffer from a comorbidity thereof, such as obesity, diabete, asthma, cancer or cardiovascular disease.

The compounds of the invention can be administered through various administration modes. The skilled person knowns how to select the most appropriate administration mode(s) depending on the compound, the disease and the subject to treat. For example, administration modes include, but are not limited to, as oral administration; administration by injection into a vein (intravenously, IV), into a muscle (intramuscularly, IM), into the space around the spinal cord (intrathecally), beneath the skin (subcutaneously, sc); sublingual administration; buccal administration; rectal administration; vaginal administration; ocular route; otic route; nasal administration; by inhalation; by nebulization in intensive mechanical circuit; cutaneous administration, either topical or systemic; transdermal administration.

In the context of the invention, the administration of the modulators of the invention is preferably performed by inhalation or by nebulization in an intensive mechanical circuit.

### Definitions in the context of the invention

As used herein, "**viral infection**" **or** "**infection with a virus**" or "**virus infection**" designates the fact that cells of an organism have been infected by at least one virus, the whole organism being said to suffer from a viral infection. In particular, "**Viral infection due to SARS-CoV-2**" or "**SARS-CoV-2 infection**" or "**SARS-CoV2 infection**" designates the fact that cells of an organism have been infected by the SARS-CoV-2 virus, the whole organism being said to suffer from said viral infection.

As used herein, the terms "**prevent**" or "**preventing**" or "**prevention**" or "**prevention of the onset of a disease**" means the reduction of the risk of appearing, of developing or of amplifying for a disease, for the causes of a disease, for the symptoms of a disease, for the effects (or consequences, preferably adverse, deleterious effects/consequences) of a disease, or any combination thereof ; and/or delaying the onset, development or amplification of a disease, the causes of a disease, the symptoms of a disease, the effects (or consequences, preferably adverse, deleterious effects/consequences) of a disease, or any combination thereof. In the present case, "preventing COVID disease" comprises reducing the likelihood that the patient undergo the switch into a severe form.

As used herein, the terms "**treat**", "**treating**", "**treatment**" and the like mean the reduction, inhibition, amelioration, stabilization and/or disappearance of a disease (or an ailment, or a condition), of the causes of a disease, of the symptoms (or signs) of a disease, of the effects (or consequences, preferably adverse, deleterious effects/consequences) of a disease (e.g. COVID such as COVID-19, and/ or symptoms associated therewith), fighting the disease, or any combination thereof. It will be appreciated that, although not precluded, treating a disorder or condition does not require that the disorder, condition or symptoms associated therewith be completely eliminated. For examples, treating results in the reduction of at least one sign or symptom of the disease or condition. Treatment includes (but is not limited to) administration of a therapy, and may be performed either prophylactically, or subsequent or the initiation of a pathologic event. Treatment can require administration of a therapy more than once.

More specifically, "**treating a betacoronavirus infection**" or "**treating COVID**" refers to fighting at least one betacoronavirus infection in a human or animal organism. Advantageously, the rate of viral infection (infectious titre) in the organism will decrease, and the betacoronavirus will completely disappear from the organism within a shorter period of time than expected without treatment. The terms "**treating a betacoronavirus infection**" or "**treating COVID**" also refer to reducing/inhibiting/ameliorating/stabilizing/making disappear, the symptoms/signs associated with a betacoronavirus infection (respiratory syndrome, kidney failure, fever, etc.). More specifically, "**treating SARS-CoV-2**" or "**treating COVID-19**" refers to fighting the SARS-CoV-2 infection in a human or animal organism. Advantageously, the rate of viral infection (infectious titre) in the organism will decrease, and the SARS-CoV-2 will completely disappear from the organism within a shorter period of time than expected without treatment. The terms "**treating SARS-CoV-2**" or "**treating COVID-19**" also refer to reducing/inhibiting/ameliorating/stabilizing/making disappear, the symptoms/signs associated with the SARS-CoV-2 infection (respiratory syndrome, kidney failure, fever, etc.).

By a "therapeutically effective amount" is meant a sufficient amount of the compound of the invention (e.g. P2Y2 receptor agonist or the inhibitor of P2X receptor activity) at a reasonable benefit/risk ratio applicable to the medical treatment. It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The term "pharmaceutically" or "pharmaceutically acceptable" refers to molecular entities and compositions that do not produce an adverse, allergic or other untoward reaction when administered to a mammal, especially a human, as appropriate. A "pharmaceutically acceptable carrier" or "excipient" refers to a non-toxic solid, semi-solid or liquid filler, diluent, encapsulating material or formulation auxiliary of any type. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The active ingredient can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The terms "**pharmaceutically acceptable salt**", as used herein, mean a salt of a compound which is pharmaceutically acceptable, as defined above, and which possesses the pharmacological activity of the corresponding compound. The pharmaceutically acceptable salts comprise:
(i) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(ii) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

In its broadest sense, a "vector" as used herein is any vehicle capable of facilitating the transfer of the antisense oligonucleotide siRNA or ribozyme nucleic acid to the cells and preferably cells expressing the targeted proteins (e.g. P2X receptor). Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUCl9, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

The invention will be further illustrated by the following figures and examples. However, these examples and figures should not be interpreted in any way as limiting the scope of the present invention.

### DESCRIPTION OF THE FIGURES

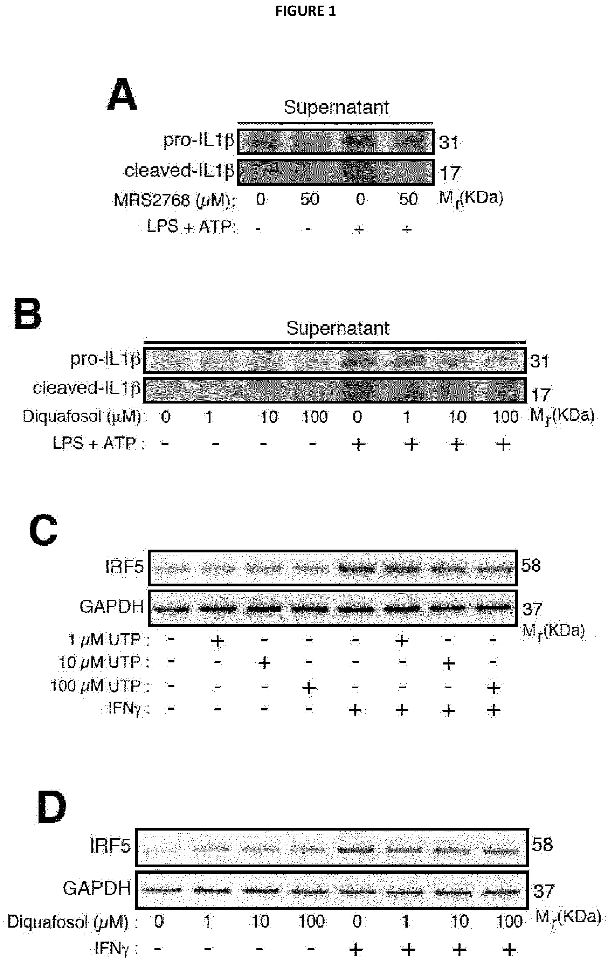

**Figure 1** shows that P2Y2 agonists inhibit IL-1β secretion detected in response to LPS+ATP stimulation and IFNγ-mediated macrophage pro-inflammatory reprogramming. (A,B) PMA-THP1 macrophages were incubated with 50 µM of MRS2768 (during 1 hour) (A) or indicated concentrations of Diquafosol (during 12 hours) (B). Cells were then stimulated with 10 ng/ml LPS (during 3 hours) and 5mM ATP (during 6 hours). IL-1β release was detected in the supernatant of treated cells by western blot. (C, D) PMA-THP1 macrophages were incubated with indicated concentrations of UTP (C) or Diquafosol (D) and stimulated with 50 ng (C) or 20 ng (D) of IFNγ during 24 (C) or 12 (D) hours. IRF5 expression was analyzed by western blot. Representative experiments of 3 independent experiments are shown.

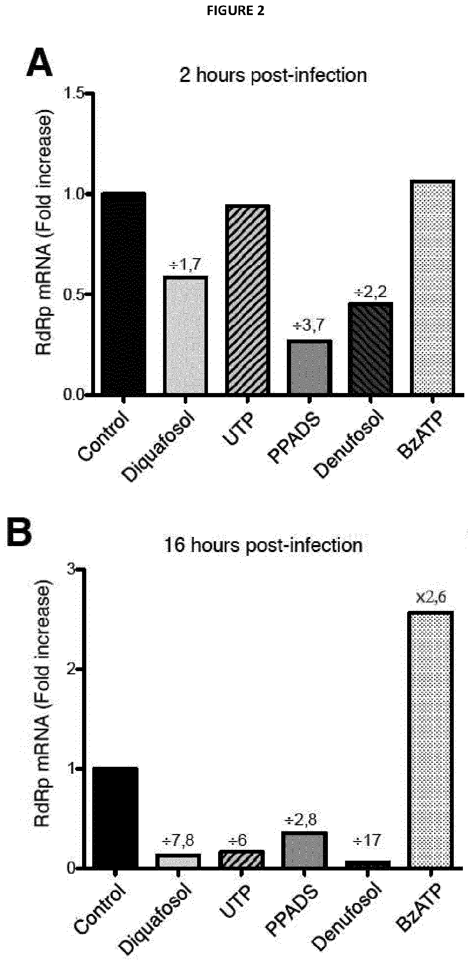

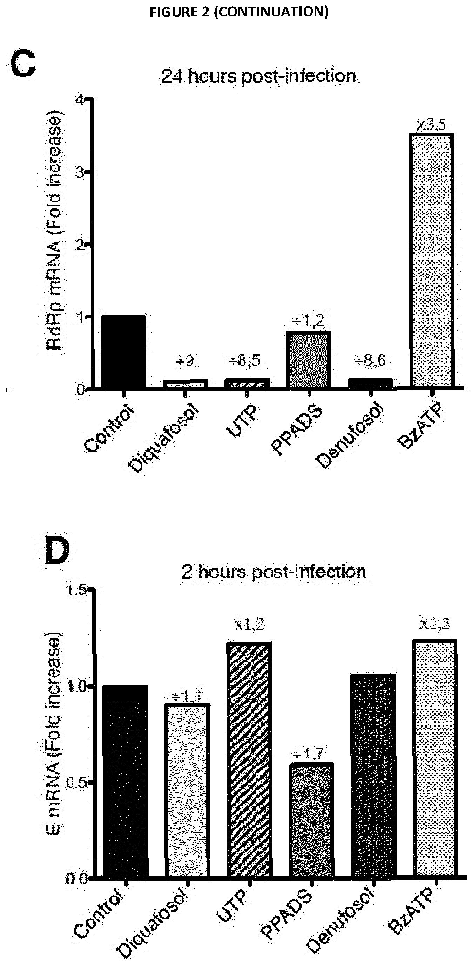

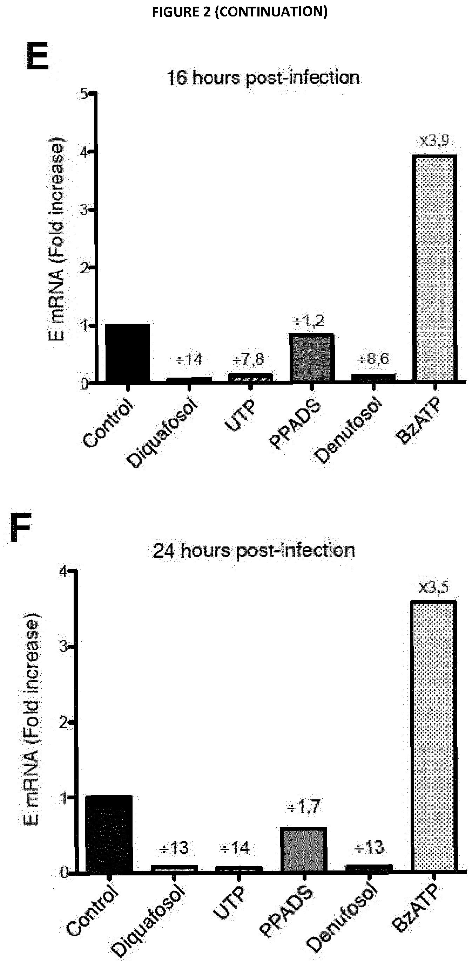

**Figure 2** demonstrates that purinergic receptors dictate viral replication. Vero E6 cells were infected with SARS-CoV-2 during 2 hours (A,D), 16 hours (B,E) and 24 hours (Figures C,F) with 500 µM UTP, 100 µM Diquafosol, 5 µM Denufosol, 50 µM PPADS or 100 µM BzATP and mRNA expression of RdRp (A-C) and E (D-F) were analyzed using quantitative RT-PCR.

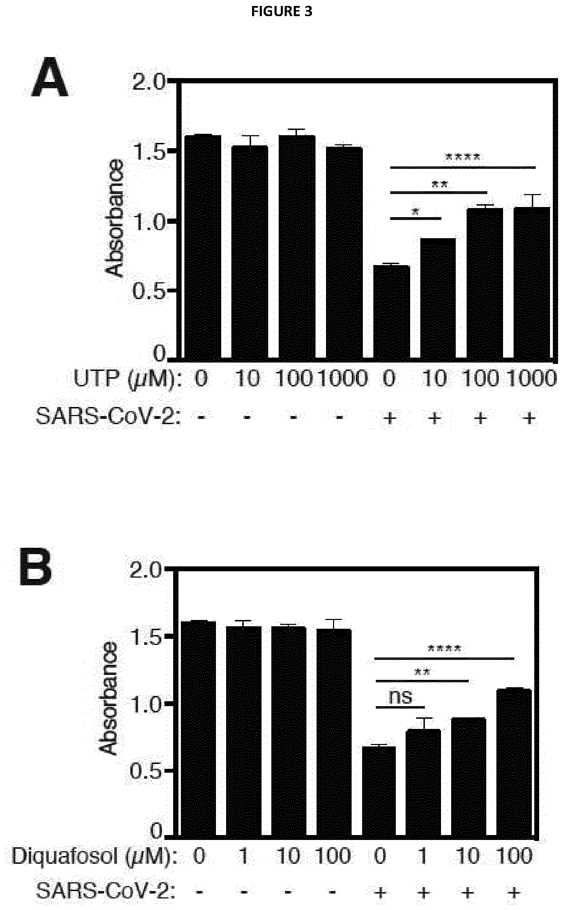

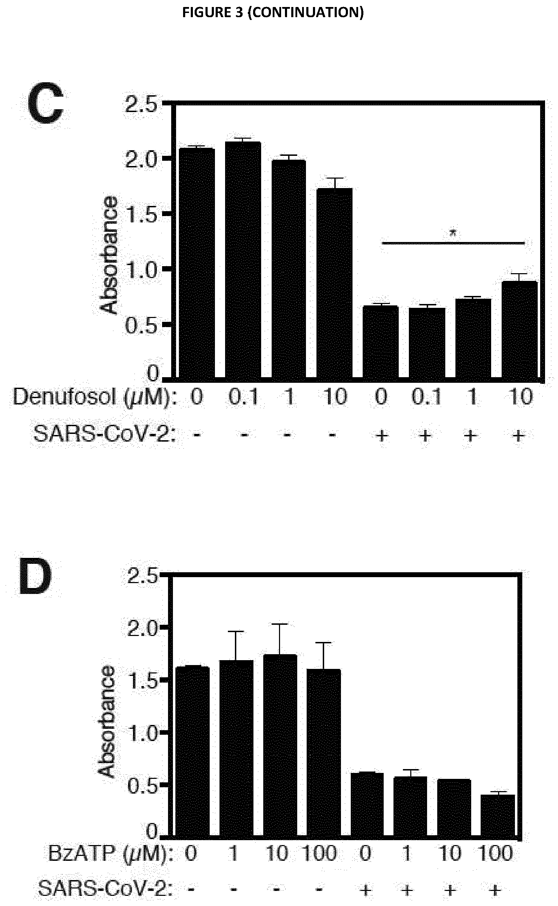

**Figure 3** disclose how purinergic receptors control cytopathogenicity elicited by SARS-CoV-2. Vero E6 cells were infected with SARS-CoV-2 during 72 hours in presence of indicated concentrations of UTP (A), Diquafosol (B), Denufosol (C) or BzATP (D) and cytopathogenicity was analyzed using MTT assay. Absorbances at 570 nm of representative experiments are shown.

### EXAMPLES

### 1. MATERIALS AND METHODS

### 1.1. Cell lines and SARS-COV-2

Monocytic THP1 cells were obtained from ATCC and were maintained in RMPI-1640-Glutamax medium supplemented with 10% heat inactivated fetal bovine serum (FBS) and 100 UI/mL penicillin-streptomycin (Life technology). THP1 macrophages were obtained by treatment for 3 hours with 100 nM phorbol-12-myristate-13-acetate (PMA, Invivogen) of THP1 monocytes and after extensive washings were let to differentiate for 72 hours before experimentation. The African green monkey kidney epithelial (Vero E6) cells were purchased from ATCC (ATCC CRL-1587) and cultured in Dulbecco's modified Eagle's medium (DMEM, Gibco, USA) with 10% fetal bovine serum (FBS), 100 U/mL penicillin, and 100µg/mL streptomycin at 37°C. All cell lines used were mycoplasma-free. SARS-CoV-2 was propagated on Vero E6 cells in a biosafety level-3 (BLS-3) laboratory. After 72 hours of infection with a multiplicity of infection of 0.2, the supernatant was collected and centrifuged during 5 minutes at 1500 rpm at 4°C to remove cellular debris. Then, supernatant was centrifuged during 20 minutes at 3000 rpm at 4°C and stored at -20°C. Viral titration was performed by determining cytopathogenic effects associated with viral infection. Cell lysis was determined using agarose-containing semi-solid medium (Björn Meyer ; Institut Pasteur). LPS, ATP, UTP, Diquafosol and Denufosol, pyridoxal phosphate-6-azophenyl-2',4'-disulfonic acid (PPDAS) and P2X7 receptor agonist 2',3'-O-(4-benzoyl-benzoyl)ATP (BzATP) were obtained from Sigma, Diquafosol from Clinisciences and Denufosol from Carbosynth.

### 1.2. Western blots

Human THP-1 cells were cultured in RPMI 1640 media, supplemented with 10% FBS and differentiated by treatment for 3 hours with 100 nM phorbol-12-myristate-13-acetate (PMA, Invivogen). After 2 days, macrophage THP-1 cells were stimulated first 3 hours with ultrapure LPS from E.coli (10 ng/ml, LPS) and then stimulated for 6 hours with ATP (5 mM, Sigma) or treated with 50 ng or 20 ng of IFNg during 24 or 12 hours as indicated. Then, supernatants and cells were collected for western blot analysis.

Cells or supernatant were lysed in appropriated buffer (250 mM NaCl, 0.1% NP-40, 5 mM EDTA, 10 mM Na3VO4, 10 mM NaF, 5 mM DTT, 3 mM Na4P2O7, 1 mM EGTA, 10 mM Glycerol phosphate, 10 mM Tris-Hcl (pH=7.5) and the protease and phosphatase inhibitors (Roche)). Equal amount of supernatant or 10-40 µg of protein extracts were run on 4-12% or 10% SDS-PAGE and transferred at 4°C onto a nitrocellulose membrane (0.2 Micron). After incubation for 2 hours at room temperature with 5% nonfat milk or BSA (Bovine Serum Albumine) in Tris-buffered saline and 0.1% Tween 20 (TBS-Tween), membranes were incubated with primary antibody at 4°C overnight. Horseradish peroxidase-conjugated goat anti-mouse or anti-rabbit (SouthernBiotech) antibodies were then incubated for 1 hour 30 minutes and revealed with the enhanced ECL detection system (GE Healthcare). The primary antibodies against IL-1β, IRF5 and GAPDH were from Abcam and Horseradish peroxidase-conjugated goat anti-rabbit (SouthernBiotech) antibodies were incubated for 1 hours and revealed with the enhanced ECL detection system (GE Healthcare). Western blots shown are representative of at least of three independent experiments.

### 1.3. Cytopathogenic assay

The cytotoxic tests were performed using Vero E6 cells. Twenty-four hours before infection, 4x10³ cells were seeded per well on 96 well plates. Cells were pretreated with indicated concentrations of UTP, Dequifosol, Denufosol and BzATP during 4 hours before infection and infected with a multiplicity of infection between 1 and 2. Viability of cells was then determined after 72 hours of infection using (bromure de 3-(4,5-dimethylthiazol-2-yl)-2,5-diphenyl tetrazolium) (MTT) assay following manufacter's instructions.

### 1.4. Quantitative RT-PCR

After infection, total cell RNA was extracted with the RNeasy minikit (QIAGEN) according to the manufacturer's instructions. RNA was recovered according to the manufacturer's instructions for total cell RNA extraction. Quantifications were performed by real-time PCR on a Light Cycler instrument (Roche Diagnostics, Meylan, France) using the second-derivative-maximum method provided by the Light Cycler quantification software, version 3.5 (Roche Diagnostics). Standard curves for Sars-Cov2 RNA quantifications were provided in the quantification kit and were generated by amplification of serial dilutions of the provided positive control. Sequences of the oligonucleotides and probes used to quantify the RdRp and E genes are given below.

For the RdRp gene the LightMix®Modular Wuhan CoV RdRP-gene530 (Cat.-No. 53-0777-96, Tib Mol biol) was used with the following primers and probe:
- RdRp SARSr-F 5' : gTgARATggTCATgTgTggCgg (SEQ ID NO:3);
- RdRp SARSr-R 5' : CARATgTTAAASACACTATTAgCATA (SEQ ID NO :4);
- RdRp SARSr-P2 5' : 6FAM-CAggTggAACCTCATCAggAgATgC-BBQ (6FAM-SEQ ID NO:5-BBQ).

For the E-gene :
- E Sarbeco P1 5' : 6FAM-ACACTAgCCATCCTTACTgCgCTTCg-BBQ (6FAM-SEQ ID NO:6-BBQ);
- E Sarbeco F 5' :ACAggTACgTTAATAgTTAATAgCgT (SEQ ID NO:7);
- E Sarbeco R 5' : ATATTgCAgCAgTACgCACACA (SEQ ID NO:8).

Conditions used for the amplification of RdRp and E genes are the same and are as follow : Reverse transcription (5 minutes), denaturation (5 minutes) and 45 cyles with the following steps (95°C, 5 s-60°C, 15 s -72°C 15 s). Results were normalized by the total amount of RNA in the sample and also reported to the condition without any compound (wild-type condition).

### 1.5. Quantification and statistical analysis

All values were expressed as the mean ± SEM of cell individual samples. Samples values were analyzed using ANOVA for multiple groups (GraphPad Prism version 6.0b; GraphPad Software).

### 2. RESULTS

### 2.1. P2Y2 agonists inhibit NLRP3-dependent IL-1β secretion and macrophage pro-inflammatory reprogramming

To study the effects of P2Y2 agonists on NLRP3 inflammasome activation, PMA-treated THP1 macrophages were analyzed for IL-1β secretion after pretreatment with 50 µM of P2Y2 agonist MRS2768 during 1 hour and stimulation with 10 ng/ml LPS during 3 hours and 5mM ATP during 6 hours (LPS+ATP). As previously described in many studies, stimulation of PMA-treated THP1 macrophages with LPS+ATP led to a robust secretion of IL-1β in the supernatant of treated cells (Figure 1A). However, treatment of cells with MRS2768 strongly inhibited the release of IL-1B (Figure 1A), thus demonstrating that the activation of P2Y2 with MRS2768 represses NLRP3 inflammasome activation.

Then, PMA-treated THP1 macrophages were simulated with LPS+ATP in presence of indicated concentrations of Diquafosol, which is a P2Y2 agonist used for the treatment of cystic fibrosis lung disease (Kellerman et al., Adv. Drug Deliv Rev (2002)) and supernatants were analyzed for IL-1β release. As shown with MRS2768 (Figure 1A), Diquafosol also impaired the release of IL-1B in the supernatant of treated macrophages (Figure 1B).

Considering that NLRP3 was also shown to contribute to macrophage pro-inflammatory reprograming (Camell et al., Semin Immunol (2015)), PMA-treated THP1 macrophages were also stimulated with IFNγ during 24 hours in presence or in absence of different concentrations of the natural P2Y2 agonist uridine-5'-triphosphate (UTP) and the expression of Interferon regulatory factor 5 (IRF5), which is a central transcription factors of macrophage pro-inflammatory reprogramming (Krausgruber et al., Nat Immunol (2011)) was determined. A decrease of IRF5 expression was detected in presence of 10 and 100 µM of UTP (Figure 1C). These results were confirmed with indicated concentrations of Diquafosol (Figure 1D), thus suggesting that P2Y2 agonists also inhibit pro-inflammatory reprogramming of macrophages. Collectively, these data demonstrate that P2Y2 agonists repress NLRP3-inflammasome activation and macrophage pro-inflammatory reprogramming.

### 2.2. Purinergic receptors control viral replication of SARS-CoV-2 and associated cytopathogenic effects

Considering that purinergic receptor P2Y2 and other purinergic receptors (such as purinergic receptor P2X7) may regulate permissivity to viral infection (Séror et al., J Exp Med (2011); Paoletti et al., Cell Rep (2019)), the impact of P2Y2 agonists (UTP, Diquafosol and Denufosol), of P2X receptor antagonist pyridoxal phosphate-6-azophenyl-2',4'-disulfonic acid (PPDAS) and of P2X7 receptor agonist 2',3'-O-(4-benzoyl-benzoyl)ATP (BzATP) was next determined on the replication of SARS-CoV-2.

African green monkey kidney epithelial (Vero E6) cells were infected with SARS-CoV-2 during 2 hours (Figures 2A and 2D), 16 hours (Figures 2B and 2E) and 24 hours (Figures 2C and 2F) in presence of 500 µM UTP, 100 µM Diquafosol, 5 µM Denufosol, 50 µM PPADS and 100 µM BzATP.

To study viral replication, mRNA expression of RNA-dependent RNA polymerase (RdRp) and E genes were analyzed using quantitative RT-PCR. The activation of the P2Y2 by UTP, Diquafosol and Denufosol strongly reduced the amount of RdRp and E mRNA in Vero cells after 16 hours (Figures 2B and 2E) and 24 hours (Figures 2C and 2F) of infection (Figures 2A-2F), as compared to control, indicating that the purinergic receptor P2Y2 acts as a restriction factor for SARS-CoV-2 infection.

Interestingly, PPDAS also repressed RdRp and E mRNA expression after 16 (Figures 2B and 2E) and 24 (Figures 2C and 2F) hours of infection and P2X7 receptor agonist 2',3'-O-(4-benzoyl-benzoyl)ATP (BzATP) strongly enhanced viral replication at these time points. These results revealed that purinergic receptors P2X and in particular, P2X7 favor the replication of SARS-CoV-2.

In parallel, the impact of purinergic modulators on cell damages elicited by SARS-CoV-2 infection was evaluated. Vero cells were infected with SARS-COV-2 during 72 hours in presence of indicated concentrations of UTP (Figure 3A), Diquafosol (Figure 3B), Denufosol (Figure 3C) and BzATP (Figure 3D). P2Y2 agonists exhibited inhibitory effects on cytopathogenicity (Figure 3A-3C) and conversely as expected, BzATP seemed to stimulate the cytopathogenic effects associated with SARS-CoV-2 infection (Figure 2D).

These results indicate that the modulation of purinergic receptor activity during SARS-CoV-2 infection affects SARS-CoV-2 pathogenicity. Altogether, these results demonstrate that purinergic receptors and extracellular nucleotides play a central role during SARS-CoV-2 infection.

## Claims

1. Modulator of the purinergic receptors or of the P2Y2R-NRLP3 related immune checkpoint, for use in treating a subject suffering from a virus-induced Acute Respiratory Distress Syndrome (ARDS).

2. Modulator for use according to claim 1, wherein said modulator is a modulator of purinergic receptors.

3. Modulator for use according to claim 1 or 2, wherein it is an agonist of a purinergic P2Y receptor or an antagonist of a purinergic P2X receptor.

4. Modulator for use according to any of claims 1-3, wherein it is an agonist of the P2Y2 receptor.

5. Modulator for use according to claim 4, wherein said agonist is chosen in the group consisting of: MRS2698, Uridine triphosphate (UTP), 4-thio-UTP, 2-thioUTP, Diquafosol, PSB1114, ATP, Denufosol, Ap4A, UTPγS, 5BrUTP and MRS2768.

6. Modulator for use according to claim 2 or 3, wherein it is an antagonist of the P2X7 receptor.

7. Modulator for use according to claim 6, wherein said antagonist is chosen in the group consisting of: PF-04905428, JNJ-47965567, Compound 16i, AZ10606120, AZD9056, GSK1482160, GSK137039A, A438079, BBG, KN62,oxATP, A74003,AACBA,A138079, A710003 et CE-224,535.

8. Modulator for use according to claim 1, wherein said modulator impair the activity of the NRLP3 inflammasome.

9. Modulator for use according to claim 8, wherein said modulator is an antagonist of NLRP3 such as Troxerutin, Tranilast or Glyburide, or a NLRP3 inflammasome inhibitor such as MCC950 or beta-Hydroxybutyrate.

10. Modulator for use according to claim 1, wherein it is able to reduce the level of extracellular nucleotides such as ATP.

11. Modulator for use according to claim 10, wherein it is the human recombinant apyrase AZD3366.

12. Modulator for use according to any of claims 1-11, wherein said subject suffers from an ARDS caused by an influenza virus, a respiratory virus, or an herpesvirus.

13. Modulator for use according to claim 12, wherein said respiratory virus is a coronavirus, preferably a betacoronavirus, more preferably a SARS-CoV-2 virus.

14. Modulator for use according to any of claim 1 to 13, wherein said subject is a human patient suffering from the COVID19 disease.
